# DEMANDE DE BREVET EUROPEEN

(11) **EP 2 962 733 A1**
(43) Date de publication de la demande: **06.01.2016**
(21) Numéro de dépôt: 15020113.5
(22) Date de dépôt: 03.07.2015
(51) Int. Cl.: A61Q 7/00, A61K 8/97, A61P 17/14

(54) **COMPOSITION COSMÉTIQUE POUR FAVORISER LA POUSSE ET/OU FREINER LA CHUTE DES CHEVEUX**

(30) Priorité: 04.07.2014 BE 201400516
(71) Demandeur: Laboratoire Puressentiel Benelux SA, 1050 Bruxelles (BE)
(72) Inventeur: Pacchioni, Marco, 1050 Bruselles (BE); Pacchioni, Isabelle, 1050 Bruselles (BE)
(74) Mandataire: Theunis, Patrick

(57) **Abrégé**

Composition cosmétique pour favoriser la pousse et/ou freiner la chute des cheveux.

L'invention se rapporte à une composition cosmétique antichute de cheveux caractérisée en ce qu'elle comprend essentiellement une association de sept racines, présente dans un complexe global à base des ingrédients suivants : extrait de ginseng rouge, huile essentielle de Gingembre, extrait de Maca, extrait de Curcuma, extrait de Harpagophytum, huile essentielle de Vétiver et extrait de Réglisse. Cette composition se présente sous forme de composition cosmétique en sérum traitant, shampoing redensifiant, friction fortifiante ou huile revitalisante.

## Description

### Domaine technique de l'invention

La présente invention concerne une composition cosmétique ayant pour effet de freiner ou de retarder la chute des cheveux ainsi que de stimuler ou de favoriser la pousse et/ou la croissance des fibres kératiniques et en particulier les cheveux.

La présente invention vise en outre à fortifier et redensifier la chevelure.

### Contexte de l'état de la technique

On recherche depuis de nombreuses années, dans l'industrie cosmétique ou pharmaceutique, des compositions permettant de supprimer ou de réduire la perte de cheveux ainsi que favoriser la croissance des cheveux.

La présente invention vise à solutionner ces problèmes grâce à l'approche multifactorielle, c'est à dire qu'on agit sur les différents facteurs responsables de la chute des cheveux.

Ce traitement s'adresse aussi bien aux hommes qu'aux femmes et quelle que soit la cause de la perte de cheveux (héréditaire ou réactionnelle).

### Objet de l'invention

L'invention vise à résoudre les problèmes de la chute des cheveux grâce à quatre produits cosmétiques, un sérum traitant, un shampoing redensifiant, une friction fortifiante et une huile revitalisante.

Dans ces quatre produits cosmétiques le sérum traitant est le produit d'attaque qui vise essentiellement à freiner la perte de cheveux, les 3 autres produits cosmétiques sont des produits complémentaires au sérum servant à fortifier les cheveux et le cuir chevelu.

Le sérum traitant intégrant cette invention a également pour but de traiter le cuir chevelu (notamment contre les pellicules et la sècheresse,...) ainsi que d'améliorer l'état des cheveux quant à la densité, l'épaisseur et l'éclat.

### Brève description de l'invention

L'invention se rapporte à une composition cosmétique comprenant des huiles essentielles et des extraits végétaux spécifiques qui, lors de leur incorporation dans un produit fini, ont pour effet de diminuer ou de retarder la perte des cheveux et de favoriser la croissance des cheveux chez l'homme (chute chronique hormonale héréditaire) comme chez la femme (chute occasionnelle ou réactionnelle : stress, fatigue, régime ou post-accouchement). Cette composition est d'origine naturelle, et basée essentiellement sur une association de sept racines, présent dans le complexe global en tant que huile essentielle, ou en tant qu'extrait végétal. Plus en particulier la composition cosmétique selon l'invention comprend l'ensemble des ingrédients suivants : extrait de ginseng rouge, huile essentielle de Gingembre, extrait de Maca, extrait de Curcuma, extrait de Harpagophytum, huile essentielle de Vétiver et extrait de Réglisse. La formule de cette composition est spécialement étudiée pour combattre efficacement toutes les causes de la chute des cheveux.

L'invention a également été décrite dans les revendications indépendantes annexes.

Des modes d'application préférés de l'invention sont décrits dans les revendications dépendantes annexes.

D'autres modes de réalisation préférés et avantages de l'invention sont décrits dans la description suivante.

### Description détaillée de l'invention

La présente invention concerne une composition cosmétique et un traitement afin de diminuer, de freiner ou de retarder la chute des cheveux ainsi que de fortifier le cuir chevelu et les cheveux.

Pour mieux comprendre l'invention, une brève description de la physiologie du cheveu sera décrite ci-dessous :
La croissance du cheveu et le renouvèlement est principalement déterminé par l'activité des follicules pileux.
Le cycle du cheveu est organisé en 3 phases, à savoir la phase anagène, la phase catagène et la phase télogène :
   Il y a d'abord la phase anagène c'est à dire la phase au cours de laquelle le cheveu s'allonge (chez l'homme cela prend 2 à 4 ans et chez la femme cela prend 3 à 6 ans).

Ensuite le cheveu passe dans la phase catagène, c'est une phase très courte et transitoire qui ne dure que quelques semaines. Le cheveu subit une involution, il s'atrophie et son implantation dermique apparait de plus en plus haute. Pour finir, la phase télogène, qui dure quelques mois, est une période de repos du cheveu et ensuite il finira par tomber.

Après cette phase de repos un nouveau cheveu est régénéré, sur place et un nouveau cycle recommence.

La chevelure se renouvelle donc en permanence.

Il est normal de perdre ses cheveux. Chaque être humain perd en moyenne entre 40 et 60 cheveux par jour en temps normal.

Ce processus de renouvèlement physique permanent subit une évolution naturelle au cours du vieillissement, les cheveux deviennent plus fins et leurs cycles plus courts. Malheureusement chez certaines personnes la nouvelle pousse de cheveux est moindre que le nombre de cheveux qui tombent. Ces personnes perdent en moyenne plus de 100 cheveux par jour.

Différentes causent peuvent entrainer une perte temporaire ou définitive des cheveux : stress, fatigue, régime, post-accouchement, ménopause etc.

Dans certaines dermatoses du cuir chevelu à caractère inflammatoire, la perte de cheveux peut également être accentuée.

L'invention se rapporte à une composition cosmétique antichute de cheveux caractérisée en ce qu'elle comprend essentiellement une association de sept racines, présente dans un complexe global à base des ingrédients énumérés ci-après.

La composition cosmétique suivant la présente invention peut comprendre un mélange spécifique d'huiles essentielles et d'extraits végétaux aux fins de commercialisation en forme de produit fini. Elle comprend les sept racines différentes décrites ci-après, en forme d'huiles essentielles et d'extraits végétaux.

Ces produits finis peuvent être utilisés dans un traitement qui pourrait solutionner les problèmes décrits supra.

Aux fins de la présente invention on entend par extrait végétal : une préparation liquide (extraits fluides et teintures), de consistance semi-solide (extraits mous ou fermes) ou solide (extraits secs), obtenues à partir de drogues végétales généralement à l'état sec. Les extraits sont définis par leur procédé de production (état de la drogue végétale, solvant, conditions d'extraction) et leurs spécifications (Définition d'après la Pharmacopée européenne). L'extraction est réalisée par un solvant approprié (généralement de l'éthanol et/ou de l'eau) à partir d'un ou plusieurs lots de drogue, qui peuvent avoir subi préalablement différents traitements comme un broyage ou encore un dégraissage.

Aux fins de la présente invention on entend par huile essentielle : une essence volatile extraite de plantes aromatiques ou d'organe de cette plante par la distillation par entraînement à la vapeur d'eau.

Le complexe des sept racines aux fins d'inclusion dans les compositions cosmétiques selon l'invention comprend les ingrédients suivants sous forme d'huiles essentielles et d'extraits végétaux :
- Ginseng rouge,
- Gingembre,
- Maca
- Curcuma,
- Harpagophytum,
- Vétiver
- Réglisse.

Selon un mode préféré de l'invention, la composition comprend un ou plusieurs des éléments suivants :
- extrait de Ginseng rouge,
- huile essentielle de Gingembre,
- extrait de Maca,
- extrait de Curcuma,
- extrait de Harpagophytum,
- huile essentielle de Vétiver
- extrait de réglisse.

Selon un mode préféré de l'invention, l'extrait des éléments cités supra est soit un extrait hydro-alcoolique, soit un extrait hydro-glycériné, soit une huile essentielle.

De plus amples détails de cet aspect de l'invention sont inclus à titre informatif not limitant pour l'invention dans les formules cadres citées infra.

Selon un mode préféré de l'invention le Ginseng rouge est le Panax Ginseng, le Gingembre est le Zingiber officinale, le Maca est le Lepidium meyenii le Curcuma est le Curcuma longa, le Harpagophytum est le Harpagophytum procumbens, le Vétiver est le Vetiveria zizanoides et le réglisse est le glycyrrhiza glabra.

Selon un mode préféré de l'invention, la concentration des racines comprises dans la composition selon l'invention, est comprise entre 0,01 % et 5 %. Selon un mode encore plus préféré, la concentration des racines est comprise entre 0,1 % et 5 %.

Sans que les inventeurs veulent se limiter à une explication scientifique des effets de l'invention : ils estiment que les effets bénéfiques de la composition cosmétique, plus spécifiquement le sérum traitant selon l'invention sont produits par une approche multifactorielle Cette approche multifactorielle résulte d'une combinaison de la composition de la présente invention sur les modes d'actions suivants :
- antihormonal ;
- stimulant de la croissance du cheveu;
- anti-inflammatoire ;
- anti-radicalaire ;
- stimulant de la circulation ;
- fortifiant et protecteur du bulbe capillaire, gainant la structure du cheveu et diminuant la chute.

Plus spécifiquement les modes d'actions précités résultent du choix des ingrédients actifs énumérés ci-après :
- antihormonal : ginseng rouge extrait (racine) ; schisandra sphenanthera extrait, huile essentielle de lemongrass, de Leptospernum petersonii de gingembre (racine), eclipta alba Teinture mère, serenoa serrulata extrait ;
- stimulant de la croissance du cheveu : ginseng rouge extrait (racine), curcuma long extrait (racine), maca extrait (racine), eclipta alba teinture mère, extrait pois ;
- anti-inflammatoire : réglisse extrait (racine), ginseng rouge extrait (racine), huile essentielle de gingembre (racine), ortie piquante extrait, Harpagophytum extrait (racine) ;
- anti-radicalaire : huile essentielle de gingembre (racine), curcuma long extrait (racine)
- stimulant de la circulation : huile essentielle de vétiver (racine), gingembre (racine), leptospermum petersoni, Lemongrass;
- fortifiant et protecteur du bulbe capillaire, gagnant la structure du cheveu et diminue la chute : maca extrait (racine), pois extrait.

L'efficacité des compositions cosmétiques selon l'invention a été testée ; ces tests se sont déroulés suivant les modes d'utilisation décrits infra.

Les modes d'utilisation des produits finis selon la présente invention en grandes lignes reprennent les modes de ces tests.

La composition utilisable selon l'invention peut en particulier comprendre une composition pour soins capillaires et peut être commercialisée sous différentes formes galéniques, notamment un sérum d'application journalier, un shampoing (bi)hebdomadaire, une lotion journalière ainsi qu'une huile également journalière.

Le sérum capillaire traitant antichute peut se présenter en forme d'une lotion hydro-alcoolique sous forme de spray. De préférence 16 pulvérisations sont appliquées, raie par raie, sur le cuir chevelu, une fois par jour en faisant pénétrer en massant du bout des doigts. Ne pas rincer. Un traitement de minimum 3 mois est recommandé.

Dans ce sérum la concentration du complexe selon l'invention est plus élevée comparée aux concentrations utilisées dans les formules de relais, tel que le shampoing, la friction ou l'huile revitalisante. La concentration du complexe dans le sérum peut se situer entre 5 et 15 %, de préférence environ 6 %.

Le shampoing redensifiant se présente sous forme de gel fluide jaune clair. Il faut appliquer le shampoing sur des cheveux humides, masser le cuir chevelu pour bien faire mousser et ensuite rincer à l'eau. A utiliser 2 à 3 fois par semaine.

La friction fortifiante se présente sous forme d'une lotion hydro alcoolique jaune claire. Appliquer sur les cheveux humides ou secs. Appliquer raie par raie la friction en quantité suffisante de façon a bien imprégner l'ensemble de la surface du cuir chevelu. Faire pénétrer en massant du bout des doigts. Ne pas rincer. Cette lotion doit être utilisée tous les jours.

L'huile revitalisante se présente sous forme de liquide limpide jaune. Il faut verser l'huile dans le creux de la main, frotter les mains l'une contre l'autre et appliquer sur la chevelure en se passant les mains dans les cheveux de façon à les coiffer. Cette huile doit également être utilisée une fois par jour.

Ci-dessous vous trouverez 4 tableaux contenant des formules cadre des quatre formes de présentations des produits cosmétiques selon invention, c'est a dire :
- le sérum traitant Anti Chute ;
- le shampoing redensifiant complément Antichute ;
- la friction fortifiante complément Antichute ;
- l'huile revitalisante complément Antichute.

Dans les tableaux ci-dessous, pour chaque formule d'application les formules cadres sont mentionnées.

Chaque tableau comporte 4 colonnes :
- la première colonne indique la dénomination INCI (CFTA),
- la deuxième colonne indique le n°CAS du produit en cause,
- la troisième colonne mentionne le n° EINECS, et
- la quatrième colonne indique les fourchettes exprimées en pourcentage de poids pour chaque produit en cause dans la composition globale.

**INCI** (nomenclature internationale des ingrédients cosmétiques), abréviation de 'International Nomenclature of Cosmetic Ingredients' a été conçue par la Cosmetic, Toiletry and Fragrance Association (CTFA) association américaine regroupant des fabricants de cosmétiques. En Europe, son utilisation est obligatoire pour les cosmétiques : tous les cosmétiques doivent faire figurer sur leur emballage la liste complète des ingrédients dans l'ordre décroissant de leur quantité et sous leur dénomination INCI

**N° EINECS :** numéro d'identification à sept chiffres se rapportant à la législation Européenne. Il doit figurer sur l'emballage des substances dangereuses.

Le numéro **CAS** d'un produit chimique, polymère, séquence biologique et alliage est son numéro d'enregistrement unique auprès de la banque des données de Chemical Abstracts Service (CAS) une division de L'American Chemical Society (ACS) Le CAS assigne ces numéros à chaque produit chimique qui a été décrit dans la documentation.

| **FORMULE CADRE SERUM TRAITANT ANTI-CHUTE** | | | | |
|---|---|---|---|---|
| | | | | |

| **Nom usuel (pour 7 rcaines)** | **Dénomination INCI** | **n°CAS** | **n° EINECS** | **%** |
|---|---|---|---|---|
| | ALCOHOL DENAT. | / | / | 50-70% |
| | | | | |
| | AQUA (WATER) | 7732-18-5 | 231-791-2 | 25-50% |
| | | | | |
| | AQUA (WATER) | 7732-18-5 | 231-791-2 | 5-10% |
| | ALCOHOL | 64-17-5 | 200-578-6 | |
| | PISUM SATIVUM (PEA) SPROUT EXTRACT | 90082-41-0 | 290-130-6 | |
| | | | | |
| Extrait glycériné maca | GLYCERIN | 56-81-5 | 200-289-5 | 5-10% |
| | LEPIDIUM MEYENII ROOT EXTRACT | | | |
| | | | | |
| | ALCOHOL | 64-17-5 | 200-578-6 | 0,1-5% |
| | AQUA (WATER) | 7732-18-5 | 231-791-2 | |
| | ECLIPTA PROSTRATA EXTRACT | 93165-22-1 | 296-907-6 | |
| | | | | |
| | AQUA (WATER) | 7732-18-5 | 231-791-2 | 0,1-5% |
| | ALCOHOL | 64-17-5 | 200-578-6 | |
| | URTICA DIOICA (NETTLE) EXTRACT | 84012-40-8 | 281-685-5 | |
| | | | | |
| | SCHIZANDRA SPHENANTHERA FRUIT EXTRACT | | | 0,1-5% |
| | AQUA (WATER) | 7732-18-5 | 231-791-2 | |
| | SODIUM BENZOATE | 532-32-1 | 208-534-8 | |
| | CITRIC ACID | 77-92-9 / 5949-29-1 | 201-069-1 | |
| | AQUA (WATER) | 7732-18-5 | 231-791-2 | 0,1-5% |
| | ALCOHOL | 64-17-5 | 200-578-6 | |
| | GLYCERIN | 56-81-5 | 200-289-5 | |
| | SERENOA SERRULATA FRUIT EXTRACT | 84604-15-9 | 283-292-4 | |
| | | | | |
| Extrait hydro-alcoolique harpagophytu m | ALCOHOL | 64-17-5 | 200-578-6 | 0,1-5% |
| | HARPAGOPHYTUM PROCUMBENS ROOT EXTRACT | 84988-65-8 | 284-853-6 | |
| | AQUA (WATER) | 7732-18-5 | 231-791-2 | |
| | | | | |
| Extrait hydro-alcoolique réglisse | GLYCYRRHIZA GLABRA (LICORICE) ROOT EXTRACT | 84775-66-6 | 283-895-2 | 0,1-5% |
| | ALCOHOL | 64-17-5 | 200-578-6 | |
| | AQUA (WATER) | 7732-18-5 | 231-791-2 | |
| | | | | |
| Extrait hydro-alcoolique ginseng | AQUA (WATER) | 7732-18-5 | 231-791-2 | 0,1-5% |
| | ALCOHOL | 64-17-5 | 200-578-6 | |
| | PANAX GINSENG ROOT EXTRACT | 84650-12-4 | 283-493-7 | |
| | | | | |
| Extrait hydro-alcoolique glyceriné curcuma | AQUA (WATER) | 7732-18-5 | 231-791-2 | 0,1-5% |
| | ALCOHOL | 64-17-5 | 200-578-6 | |
| | GLYCERIN | 56-81-5 | 200-289-5 | |
| | CURCUMA LONGA (TURMERIC) ROOT EXTRACT | 84775-52-0 | 283-882-1 | |
| | | | | |
| Huile essentielle vetyver | VETIVERIA ZIZANOIDES ROOT OIL | 8016-96-4 / 84238-29-9 | 282-490-8 | 0,01-5% |
| | | | | |
| | CYMBOPOGON FLEXUOSUS OIL | 91844-92-7 | 295-161-9 | 0,01-5% |
| | | | | |
| Huile essentielle gingembre | ZINGIBER OFFICINALE (GINGER) ROOT OIL | 8007-08-7 | / | 0,01-5% |
| | | | | |
| | MENTHA PIPERITA (PEPPERMINT) OIL | 8006-90-4 / 84082-70-2 | -/282-015-4 | 0,01-5% |
| | | | | |
| | LEPTOSPERMUM PETERSONII OIL | 85085-43-4 | 285-372-4 | 0,01-5% |

| **FORMULE CADRE SHAMPOONIG REDENSIFIANT COMPLEMENT ANTI-CHUTE** | | | | |
|---|---|---|---|---|
| | | | | |

| **Nom usuel (pour 7 racines)** | **Dénomination INCI** | **n°CAS** | **n° EINECS** | **%** |
|---|---|---|---|---|
| | AQUA (WATER) | 7732-18-5 | 231-791-2 | 50-70% |
| | | | | |
| | SODIUM LAURYL SULFATE | 151-21-3 | 205-788-1 | 10-25% |
| | AQUA (WATER) | 7732-18-5 | 231-791-2 | |
| | | | | |
| | SODIUM COCOAMPHOACETATE | 90387-76-1 | 291-352-6 | 5-10% |
| | AQUA (WATER) | 7732-18-5 | 231-791-2 | |
| | SODIUM CHLORIDE | 7647-14-5 | 231-598-3 | |
| | | | | |
| | GLYCERIN | 56-81-5 | 200-289-5 | 5-10% |
| | SODIUM PCA | 28874-51-3 | 249-277-1 | |
| | AQUA (WATER) | 7732-18-5 | 231-791-2 | |
| | | | | |
| | SODIUM COCOYL GLUTAMATE | 68187-32-6 | 269-087-2 | 0,1-5% |
| | AQUA (WATER) | 7732-18-5 | 231-791-2 | |
| | SODIUM CHLORIDE | 7647-14-5 | 231-598-3 | |
| | | | | |
| | AQUA (WATER) | 7732-18-5 | 231-791-2 | 0,1-5% |
| | CAPRYLYL/CAPRYL GLUCOSIDE | 68515-73-1 | 500-220-1 | |
| | | | | |
| | SODIUM CHLORIDE | 7647-14-5 | 231-598-3 | 0,1-5% |
| | | | | |
| | SODIUM BENZOATE | 532-32-1 | 208-534-8 | 0,1-5% |
| | | | | |
| Extrait glycériné maca | GLYCERIN | 56-81-5 | 200-289-5 | 0,1-5% |
| | LEPIDIUM MEYENII ROOT EXTRACT | / | / | |
| | | | | |
| | ACETUM (VINEGAR) | 90132-02-8 | 290-419-7 | 0,1-5% |
| | | | | |
| | CITRIC ACID | 77-92-9 / 5949-29-1 | 201-069-1 | 0,1-5% |
| | | | | |
| | HYDROXYPROPYL GUAR HYDROXYPROPYLTRI MONIUM CHLORIDE | 71329-50-5 | / | 0,1-5% |
| | | | | |
| Extrait hydro-alcoolique ginseng | AQUA (WATER) | 7732-18-5 | 231-791-2 | 0,01-5% |
| | ALCOHOL | 64-17-5 | 200-578-6 | |
| | PANAX GINSENG ROOT EXTRACT | 84650-12-4 | 283-493-7 | |
| | | | | |
| | CEDRUS ATLANTICA WOOD OIL | 92201-55-3 | 295-985-9 | 0,01-5% |
| | | | | |
| Extrait hydro-alcoolique réglisse | GLYCYRRHIZA GLABRA (LICORICE) ROOT EXTRACT | 84775-66-6 | 283-895-2 | 0,01-5% |
| | ALCOHOL | 64-17-5 | 200-578-6 | |
| | AQUA (WATER) | 7732-18-5 | 231-791-2 | |
| | | | | |
| | CITRUS AURANTIUM DULCIS (ORANGE) PEEL OIL | 8008-57-9 | | 0,01-5% |
| | | | | |
| | CITRUS LIMON (LEMON) PEEL OIL | 8008-56-8 / 84929-31-7 | - / 284-515-8 | 0,01-5% |
| | | | | |
| Extrait hydro-alcoolique harpagophytu m | ALCOHOL | 64-17-5 | 200-578-6 | 0,01-5% |
| | HARPAGOPHYTUM PROCUMBENS ROOT EXTRACT | 84988-65-8 | 284-853-6 | |
| | AQUA (WATER) | 7732-18-5 | 231-791-2 | |
| | | | | |
| Extrait hydro-alcoolique glyceriné curcuma | AQUA (WATER) | 7732-18-5 | 231-791-2 | 0,01-5% |
| | ALCOHOL | 64-17-5 | 200-578-6 | |
| | GLYCERIN | 56-81-5 | 200-289-5 | |
| | CURCUMA LONGA (TURMERIC) ROOT EXTRACT | 84775-52-0 | 283-882-1 | |
| | | | | |
| | LAVANDULA ANGUSTIFOLIA (LAVENDER) OIL | 8000-28-0 / 90063-37-9 | - / 289-995-2 | 0,01-5% |
| | | | | |
| | MELALEUCA VIRIDIFLORA LEAF OIL | 132940-73-9 | 310-217-5 | 0,01-5% |
| | | | | |
| | ROSMARINUS OFFICINALIS (ROSEMARY) LEAF OIL | 84604-14-8 / 8000-25-7 | 283-291-9 | 0,01-5% |
| | | | | |
| | THYMUS VULGARIS (THYME) FLOWER/LEAF OIL | 84929-51-1 / 8007-46-3 | 284-535-7/- | 0,001-5% |
| | | | | |
| Huile essentielle vetyver | VETIVERIA ZIZANOIDES ROOT OIL | 8016-96-4/ 84238-29-9 | 282-490-8 | 0,001-5% |
| | | | | |
| Huile essentielle gingembre | ZINGIBER OFFICINALE (GINGER) ROOT OIL | 8007-08-7 | / | 0,001-5% |
| | | | | |
| | SODIUM HYDROXIDE | 1310-73-2 | 215-185-5 | 0-5% |
| | AQUA (WATER) | 7732-18-5 | 231-791-2 | |

| **FORMULE CADRE FRICTION FORTIFIANTE COMPLEMENT ANTI-CHUTE** | | | | |
|---|---|---|---|---|
| | | | | |

| **Nom usuel (pour 7 racines)** | **Dénomination INCI (CTFA)** | **n°CAS** | **n° EINECS** | **%** |
|---|---|---|---|---|
| | ALCOHOL DENAT. | / | | 50-70% |
| | | | | |
| | AQUA (WATER) | 7732-18-5 | 231-791-2 | 30-50% |
| | | | | |
| Extrait glycériné maca | GLYCERIN | 56-81-5 | 200-289-5 | 0,1-5% |
| | LEPIDIUM MEYENII ROOT EXTRACT | / | / | |
| | | | | |
| | AQUA (WATER) | 7732-18-5 | 231-791-2 | 0,1-5% |
| | ALCOHOL | 64-17-5 | 200-578-6 | |
| | PISUM SATIVUM (PEA) SPROUT EXTRACT | 90082-41-0 | 290-130-6 | |
| | | | | |
| | AQUA (WATER) | 7732-18-5 | 231-791-2 | 0,1-5% |
| | ALCOHOL | 64-17-5 | 200-578-6 | |
| | GLYCERIN | 56-81-5 | 200-289-5 | |
| | SERENOA SERRULATA FRUIT EXTRACT | 84604-15-9 | 283-292-4 | |
| | | | | |
| | ALCOHOL | 64-17-5 | 200-578-6 | 0,1-5% |
| | AQUA (WATER) | 7732-18-5 | 231-791-2 | |
| | ECLIPTA PROSTRATA EXTRACT | 93165-22-1 | 296-907-6 | |
| | | | | |
| Extrait hydro-alcoolique ginseng | AQUA (WATER) | 7732-18-5 | 231-791-2 | 0,01-5% |
| | ALCOHOL | 64-17-5 | 200-578-6 | |
| | PANAX GINSENG ROOT EXTRACT | 84650-12-4 | 283-493-7 | |
| | | | | |
| | AQUA (WATER) | 7732-18-5 | 231-791-2 | 0,01-5% |
| | ALCOHOL | 64-17-5 | 200-578-6 | |
| | URTICA DIOICA (NETTLE) EXTRACT | 84012-40-8 | 281-685-5 | |
| | | | | |
| Extrait hydro-alcoolique réglisse | GLYCYRRHIZA GLABRA (LICORICE) ROOT EXTRACT | 84775-66-6 | 283-895-2 | 0,01-5% |
| | ALCOHOL | 64-17-5 | 200-578-6 | |
| | AQUA (WATER) | 7732-18-5 | 231-791-2 | |
| | | | | |
| Extrait hydro-alcoolique harpagophy tum | ALCOHOL | 64-17-5 | 200-578-6 | 0,01-5% |
| | HARPAGOPHYTUM PROCUMBENS ROOT EXTRACT | 84988-65-8 | 284-853-6 | |
| | AQUA (WATER) | 7732-18-5 | 231-791-2 | |
| | | | | |
| Extrait hydro-alcoolique glyceriné curcuma | AQUA (WATER) | 7732-18-5 | 231-791-2 | 0,01-5% |
| | ALCOHOL | 64-17-5 | 200-578-6 | |
| | GLYCERIN | 56-81-5 | 200-289-5 | |
| | CURCUMA LONGA (TURMERIC) ROOT EXTRACT | 84775-52-0 | 283-882-1 | |
| | | | | |
| | SCHIZANDRA SPHENANTHERA FRUIT EXTRACT | / | / | 0,01-5% |
| | AQUA (WATER) | 7732-18-5 | 231-791-2 | |
| | SODIUM BENZOATE | 532-32-1 | 208-534-8 | |
| | CITRIC ACID | 77-92-9 / 5949-29-1 | 201-069-1 | |
| | | | | |
| Huile essentielle vetyver | VETIVERIA ZIZANOIDES ROOT OIL | 8016-96-4 / 84238-29-9 | 282-490-8 | 0,01-5% |
| | | | | |
| | CYMBOPOGON FLEXUOSUS OIL | 91844-92-7 | 295-161-9 | 0,01-5% |
| | | | | |
| Huile essentielle gingembre | ZINGIBER OFFICINALE (GINGER) ROOT OIL | 8007-08-7 | / | 0,01-5% |
| | | | | |
| | MENTHA PIPERITA (PEPPERMINT) OIL | 8006-90-4 / 84082-70-2 | - / 282-015-4 | 0,01-5% |
| | | | | |
| | LEPTOSPERMUM PETERSONII OIL | 85085-43-4 | 285-372-4 | 0,01-5% |

| **FORMULE CADRE HUILE REVITALISANTE COMPLEMENT ANTI-CHUTE** | | | | |
|---|---|---|---|---|
| | | | | |

| **Nom usuel (pour 7 racines)** | **Dénomination INCI** | **n°CAS** | **n° EINECS** | **%** |
|---|---|---|---|---|
| | SESAMUM INDICUM (SESAME) SEED OIL | 8008-74-0 | 232-370-6 | 70-95% |
| | | | | |
| | ARGANIA SPINOSA KERNEL OIL | 223747-87-3 | / | 0,1-5% |
| | | | | |
| | CEDRUS ATLANTICA WOOD OIL | 92201-55-3 | 295-985-9 | 0,1-5% |
| | | | | |
| | CITRUS AURANTIUM DULCIS | 8008-57-9 | / | 0,1-5% |
| | (ORANGE) PEEL OIL | | | |
| | | | | |
| Extrait huileux maca | HELIANTHUS ANNUUS (SUNFLOWER) SEED OIL | 8001-21-6 | 232-273-9 | 0,1-5% |
| | LEPIDIUM MEYENII ROOT EXTRACT | / | / | |
| | | | | |
| | CITRUS LIMON (LEMON) PEEL OIL | 8008-56-8 / 84929-31-7 | - / 284-515-8 | 0,1-5% |
| | | | | |
| | LAVANDULA ANGUSTIFOLIA (LAVENDER) OIL | 8000-28-0 / 90063-37-9 | - / 289-995-2 | 0,1-5% |
| | | | | |
| | MELALEUCA VIRIDIFLORA LEAF OIL | 132940-73-9 | 310-217-5 | 0,1-5% |
| | | | | |
| | ROSMARINUS OFFICINALIS (ROSEMARY) LEAF OIL | 84604-14-8 / 8000-25-7 | 283-291-9 | 0,1-5% |
| | | | | |
| | HELIANTHUS ANNUUS (SUNFLOWER) SEED OIL | 8001-21-6 | 232-273-9 | 0,1-5% |
| | TOCOPHEROL | 1406-66-2 / 10191-41-0/ 2074-53-5 / 59-02-9 / 148-03-8/ 119-13-1/ 54-28-4 | / | |
| | | | | |
| Extrait huileux ginseng | HELIANTHUS ANNUUS (SUNFLOWER) SEED OIL | 8001-21-6 | 232-273-9 | 0,01-5% |
| | PANAX GINSENG ROOT EXTRACT | 84650-12-4 | 283-493-7 | |
| | | | | |
| | THYMUS VULGARIS (THYME) FLOWER/LEAF OIL | 84929-51-1 / 8007-46-3 | 284-535-7/- | 0,01-5% |
| | | | | |
| Extrait huileux réglisse | HELIANTHUS ANNUUS (SUNFLOWER) SEED OIL | 8001-21-6 | 232-273-9 | 0,01-5% |
| | GLYCYRRHIZA GLABRA (LICORICE) ROOT EXTRACT | 84775-66-6 | 283-895-2 | |
| | | | | |
| Extrait huileux harpagophytum | HELIANTHUS ANNUUS (SUNFLOWER) SEED OIL | 8001-21-6 | 232-273-9 | 0,01-5% |
| | HARPAGOPHYTU M PROCUMBENS ROOT EXTRACT | 84988-65-8 | 284-853-6 | |
| | | | | |
| Extrait huileux curcuma | CAPRYLIC/CAPRI C TRIGLYCERIDE | 73398-61-5 / 65381-09-1 | | 0,01-5% |
| | CURCUMA LONGA (TURMERIC) ROOT EXTRACT | 84775-52-0 | 283-882-1 | |
| | | | | |
| Huile essentielle vetyver | VETIVERIA ZIZANOIDES ROOT OIL | 8016-96-4 / 84238-29-9 | / | 0,01-5% |
| | | | | |
| | CYMBOPOGON FLEXUOSUS OIL | 91844-92-7 | 295-161-9 | 0,01-5% |
| | | | | |
| Huile essentielle gingembre | ZINGIBER OFFICINALE (GINGER) ROOT OIL | 8007-08-7 | / | 0,01-5% |
| | | | | |
| | MENTHA PIPERITA (PEPPERMINT) OIL | 8006-90-4 / 84082-70-2 | -/282-015-4 | 0,01-5% |
| | | | | |
| | LEPTOSPERMUM PETERSONII OIL | 85085-43-4 | 285-372-4 | 0,01-5% |

Des tests ont été effectués pour déterminer l'efficacité antichute du sérum capillaire sur la formule pilaire (proportion de cheveux en phase anagène et télogène et la densité des cheveux) après quelques semaines d'utilisation. Ces évaluations ont été effectuées
- par inspection visuelle de photo trichogrammes;
- par cotation clinique effectuée par un dermatologue :
   o évaluation de l'état du cuir chevelu (pellicules, sècheresse, ...) ;
   o évaluation de l'état des cheveux (densité, épaisseur, éclat)
- par des tests d'usage (auto-évaluation des volontaires).

L'évaluation par photo trichogramme se fait de la façon suivante :
Une zone de 2 cm² du cuir chevelu est rasée au début du test (jour 0, J0) et une macro photo est prise du cuir chevelu. Après 2 jours de traitement la même zone est à nouveau photographiée. Puis on détermine le nombre de cheveux en croissance (anagène, A) et en non croissance (télogène, T). Sur la base des résultats obtenus on obtient le coefficient de croissance A/T. De telles photo trichogrammes ont été prises du cuir chevelu de 30 sujets, 20 femmes et 10 hommes, et à 3 instants : J0, après 1,5 mois et après 3 mois.

L'inclusion des volontaires pour les tests décrits supra a été décidée sur la base de l'échelle d'Hamilton pour les hommes et l'échelle de Ludwig pour les femmes, échelle connue en soi pour l'homme du métier.

Pour les hommes, la formation de la calvitie suit dans 80% des cas le même schéma. L'échelle d'Hamilton-Norwood le décrit.

Hamilton et Norwood ont développé cette échelle de la chute des cheveux d'origine héréditaire en 1951 et 1975. Elle est valable aujourd'hui encore et décrit un processus toujours identique qui conduit à la calvitie. Celle échelle sert au diagnostic médical pour estimer la vitesse et la gravité de la chute des cheveux.

Pour les femmes, on utilise généralement la classification Ludwig.

Dans ce cas d'espèce les critères d'inclusion des volontaires pour les tests d'efficacité sont basés sur ces échelles Hamilton-Norwood et Ludwig :
- stade 3 ou 4 sur l'échelle d'Hamilton pour les hommes, et/ou
- stade 1 à 2 sur l'échelle Ludwig pour les femmes.

Egalement, les critères d'inclusion des personnes volontaires pour les tests comprenaient les caractéristiques suivantes :
- densité de cheveux ≥ 150 cheveux par cm² au niveau de la zone de rasage ;
- proportion de cheveux en phase télogène ≥ 20% chez les hommes et ≥ 15% chez les femmes.

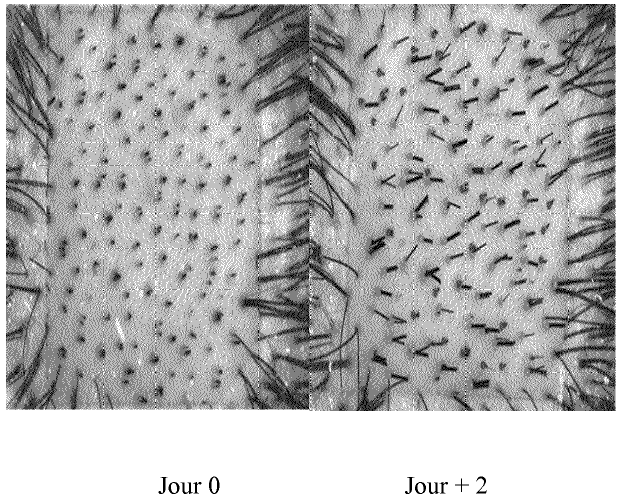

Les résultats des tests prouvent l'efficacité de l'utilisation des compositions selon l'invention :
- diminution de la chute des cheveux;
- stimulation de la croissance des cheveux;
- renforcement de l'ancrage du cheveu;
- augmentation de la densité capillaire ;
- réduction des pellicules.

La composition selon l'invention peut contenir des adjuvants habituels de façon connue en soi pour l'homme du métier dans le domaine d'application cosmétique, tels que les gélifiants ou épaississants hydrophiles ou lipophiles, les additifs hydrophiles ou lipophiles, les solvants, les charges, les conservateurs, etc.

Comme solvant utilisable dans les compositions selon l'invention, nous citons l'alcool notamment l'éthanol.

## Revendications

1. Composition cosmétique antichute de cheveux **caractérisée en ce qu'**elle comprend un complexe d'huiles essentielles et/ou d'extraits végétaux des ingrédients suivants :
- Ginseng rouge,
- Gingembre,
- Maca,
- Curcuma,
- Harpagophytum,
- Vétiver
- Réglisse.

2. Composition suivant la revendication 1, **caractérisée en ce que** le complexe comprend les ingrédients suivants :
- extrait de ginseng rouge,
- huile essentielle de Gingembre,
- extrait de Maca,
- extrait de Curcuma,
- extrait de Harpagophytum,
- huile essentielle de Vétiver,
- extrait de Réglisse.

3. Composition suivant la revendication 1 ou 2, **caractérisée en ce qu'**elle comprend en outre un milieu cosmétologiquement acceptable constitué d'eau ou d'au moins un solvant choisi parmi les solvants organiques hydrophiles, les solvants organiques lipophiles, les solvants organiques amphiphiles et leurs mélanges.

4. Composition suivant une quelconque des revendications précédentes **caractérisée en ce que** le complexe d'huiles essentielles et d'extraits végétaux représente entre 5 et 15 % en poids de la composition.

5. Composition selon une des revendications précédentes sous forme de sérum traitant, shampoing redensifiant, friction fortifiante ou huile revitalisante.

6. Composition selon une quelconque des revendications précédentes dans laquelle le Ginseng rouge est le Panax ginseng.

7. Composition selon une quelconque des revendications précédentes dans laquelle le Gingembre est le Zingiber officinale.

8. Composition selon une quelconque des revendications précédentes dans laquelle le Maca est le Lepidium meyenii.

9. Composition selon une quelconque des revendications précédentes dans laquelle le Curcuma est le Curcuma longa.

10. Composition selon une quelconque des revendications précédentes dans laquelle le Harpagophytum est le Harpagophytum procumbens.

11. Composition selon une quelconque des revendications précédentes dans laquelle le Vétiver est le Vetiveria zizanoides.

12. Composition selon une quelconque des revendications précédentes dans laquelle le réglisse est le Glycyrrhiza glabra.

13. Utilisation d'une composition cosmétique selon une quelconque des revendications précédentes pour traiter la chute des cheveux et/ou pour stimuler la croissance des cheveux.

14. Utilisation selon la revendication 13 **caractérisée en ce que** la composition est destinée à une application sur le cuir chevelu.
